# EUROPEAN PATENT SPECIFICATION

(11) **EP 2 822 628 B1**
(45) Date of publication and mention of the grant of the patent: **14.11.2018**
(21) Application number: 13711235.5
(22) Date of filing: 08.03.2013
(51) Int. Cl.: A61M 25/00, A61M 25/10

(54) **BALLOON CATHETER WITH EXPANDABLE SHAFT**
BALLONKATHETER MIT EXPANDIERBAREM SCHAFT
CATHÉTER À BALLONNET ÉQUIPÉ D'UNE TIGE EXTENSIBLE

(30) Priority: 09.03.2012 NL 2008460; 09.03.2012 US 201261608932 P
(43) Date of publication of application: 14.01.2015
(73) Proprietor: Clearstream Technologies Limited, Enniscorthy, County Wexford (IE)
(72) Inventor: JENSEN, Angela, Kay, Tempe, AZ 85281 (US); SCHAFFER, Andrew, Tempe, AZ 85281 (US)
(74) Representative: Hoffmann Eitle
(86) International application number: PCT/US2013/029995
(87) International publication number: WO 2013/134704

(56) References cited:
- EP-A1- 0 850 659
- WO-A2-2007/054364
- US-A1- 2006 287 665
- US-A1- 2009 171 278
- US-B1- 6 540 721

## Description

The following U.S. Patent Applications are disclosing a similar device; 61/608,852; 61/608,859; 61/608,862; 61/608,897; 61/608,902; 61/608,908; 61/608,913; 61/608,917; 61/608,927; 61/608,932; 61/608,941; and 61/747,444.

### Technical Field

This disclosure relates generally to catheters for performing medical procedures, such as balloon angioplasty and, more particularly, to a balloon catheter including an expandable shaft, such as in the longitudinal direction, for helping to ensure the proper alignment between a radiopaque identifier and a portion of the balloon, such as the working surface. Such a device is disclosed in the US20060287665.

### Background of the Invention

Balloons are routinely used to resolve or address flow restrictions or perhaps even complete blockages in tubular areas of the body, such as arteries or veins. In many clinical situations, the restrictions are caused by hard solids, such as calcified plaque, and require the use of high pressures to compact such blockages. Commercially available balloons employ complex technology to achieve high pressure requirements without sacrificing the profile of the balloon. Besides high pressure requirements, the balloons should also be resistant to puncture, easy to track and push, and present a low profile, especially when used for angioplasty.

In clinical practice, angioplasty balloons are expanded from a deflated, folded state to an expanded state within a vessel to treat a target area, such as a portion of the circumferential inner wall I of a blood vessel V, as shown in Figures 1 and 2. The inflation of a balloon 12 with wall 28 is traditionally completed using an X-ray contrast agent CM along dimension DX to provide better visibility under X-ray or other form of radiography R during the interventional procedure, as illustrated in Figures 3 and 3a (which shows the intensity measured by a fluoroscope detector plate, FDP). Typically, a 70/30 percent mixture of contrast agent and saline is used to inflate the balloon during an angioplasty procedure.

In general, a desirable goal is to reduce inflation and deflation times required for balloons without sacrificing the profile of the balloons, especially for large volume balloons (which can require up to two minutes of inflation/deflation times with the contrast agent). Because of its relatively high viscosity, it would also be desirable to eliminate, or at least reduce the amount of, the contrast agent used in inflation/deflation of the balloons. The use of contrast agent prolongs the inflation/deflation times and also poses the risk of iodine exposure to patients sensitive to iodine. In this regard, a non-radiopaque substance could be used in lieu of the contrast agent, such as for example saline or carbon dioxide, but such substances are invisible during X-ray imaging, and thus do not enhance visibility.

Furthermore, the physician performing the angioplasty procedure should be able to locate the position of the uninflated balloon with accuracy, so that the balloon will be properly positioned once inflated. This is conventionally accomplished by attaching marker bands on the catheter shaft in the region corresponding to the balloon working surface. This "working surface" is the surface along the portion of the balloon that is used to achieve the desired treatment effect, such as contacting the calcified plaque (which surface in the case of a balloon having conical or tapering sections at the proximal and distal ends is typically co-extensive with a generally cylindrical barrel section).

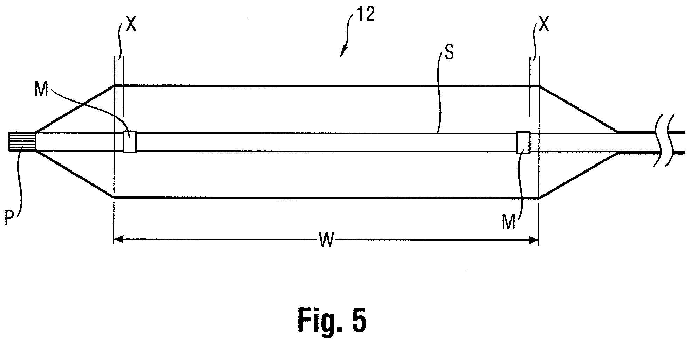

Misalignment of the marker bands during placement along the shaft sometimes results in their failure to correspond precisely to the extent of the working surface, as is shown in Figure 5 (note misalignment amount X between each interior marker band M carried by shaft S and working surface W of balloon 12, which also typically includes a radiopaque tip P at the distal end). Even upon exercising great care to position the markers properly on the underlying shaft in alignment with anticipated boundaries of the working surface when the balloon is inflated, there remains a tendency for mismatch due to several possible factors. One such factor may be the tolerance stack-ups arising as a consequence of the affixation of the balloon to the distal end of the catheter shaft. The balloon also has a tendency to grow in the longitudinal direction when inflated, especially with large and particularly long balloons. Another factor is the tendency of the portion of the catheter shaft within the balloon to bend or flex during inflation. This may lead to misalignment between radiopaque markers fixed to the shaft and the working surface.

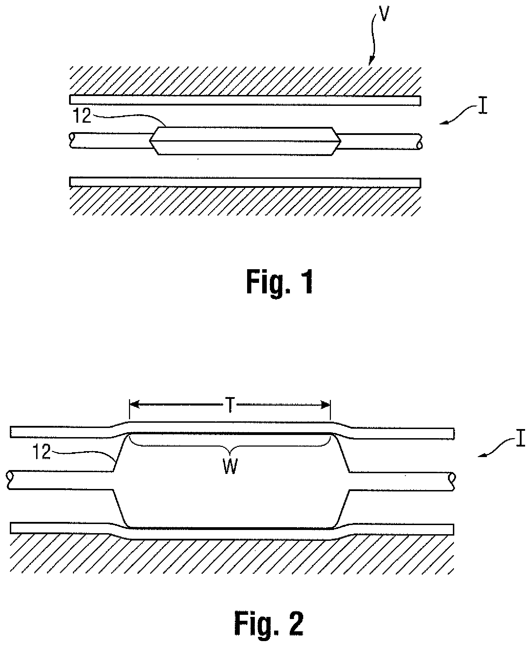

Whatever the cause, the resulting misalignment may prevent the clinician from accurately identifying the location of the working surface of the balloon during an interventional procedure. This may lead to a geographic misplacement, or "miss," of the intended contact between the target area T and the working surface W of the balloon 12 (see Figure 2). It is especially desirable to avoid such an outcome when the balloon is designed to deliver a payload (such as a drug, stent, or both) or a working element to a specified location within the vasculature, since a miss may prolong the procedure (such as, for example, by requiring redeployment of the balloon 12 or the use of another balloon catheter in the case of a drug coated balloon).

Accordingly, the need is identified for a balloon for which the working surface may be identified during an interventional procedure with enhanced precision. The solution would take into account the possible mismatch between fixed locations on the catheter shaft and the balloon to define the working surface, and may also provide for an enhanced manner of providing the inflation fluid to the balloon. Overall, procedural efficiency would be enhanced without remarkably increasing cost or complexity, and in a manner that can be applied to many existing catheter technologies without extensive modification.

### Summary of the Invention

An object of the disclosure is to provide a balloon catheter including an expandable shaft for helping to ensure proper alignment, as further defined in claim 1.

### Brief Description of the Drawings

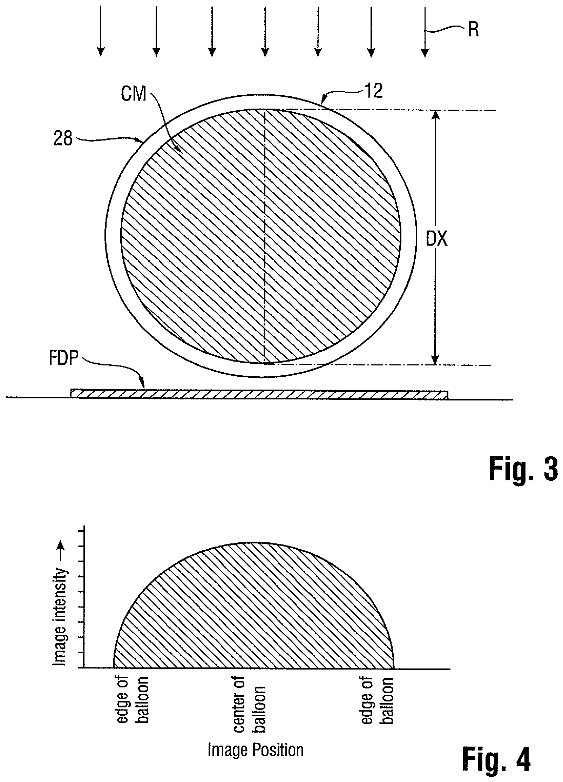

Figures 1-9 are illustrative of the background of the invention;

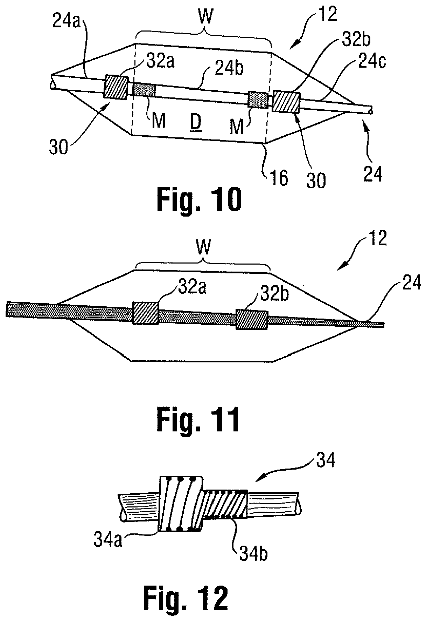

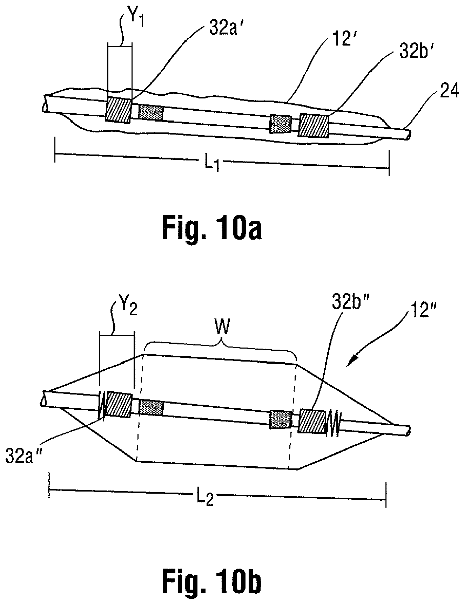

Figure 10 illustrates a first embodiment according to the disclosure;
Figures 10a and 10b illustrate additional aspects of the disclosure;
Figure 11 illustrates a second embodiment according to the disclosure;
Figure 12 illustrates a third embodiment according to the disclosure;

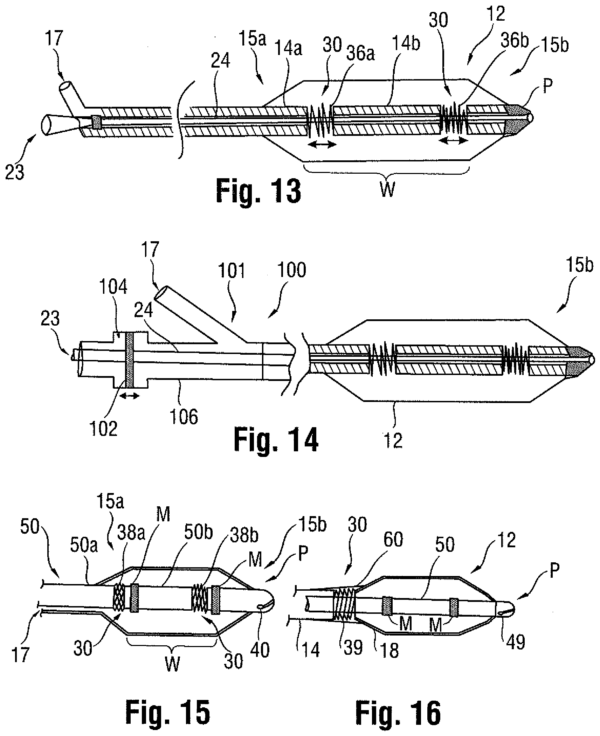

Figure 13 illustrates a fourth embodiment according to the disclosure;
Figure 14 illustrates a fifth embodiment according to the disclosure;
Figure 15 illustrates a sixth embodiment according to the disclosure;
Figure 16 illustrates a seventh embodiment according to the disclosure;

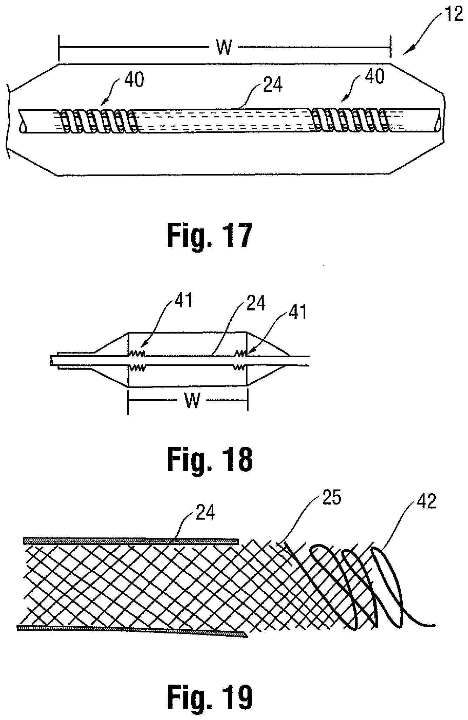

Figure 17 illustrates an eighth embodiment according to the disclosure;
Figure 18 illustrates a ninth embodiment according to the disclosure; and
Figure 19 illustrates a tenth embodiment according to the disclosure.

### Modes for Carrying Out the Invention

The description provided below and in regard to the figures applies to all embodiments unless noted otherwise, and features common to each embodiment are similarly shown and numbered.

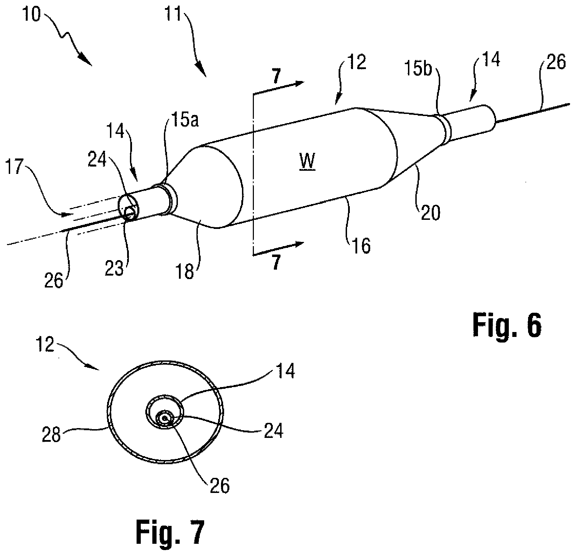

Provided is a catheter 10 having a distal portion 11 with a balloon 12 mounted on a catheter tube 14. Referring to Figures 6, 7, and 8, the balloon 12 has an intermediate section 16, or "barrel," and end sections 18, 20. In one embodiment, the end sections 18, 20 reduce in diameter to join the intermediate section 16 to the catheter tube 14 (and thus sections 18, 20 are generally termed cones or cone sections). The balloon 12 is sealed at balloon ends (proximal end 15a and distal end 15b) on the cone sections 18, 20 to allow the inflation of the balloon 12 via one or more inflation lumens 17 extending within catheter tube 14 and communicating with the interior of the balloon 12.

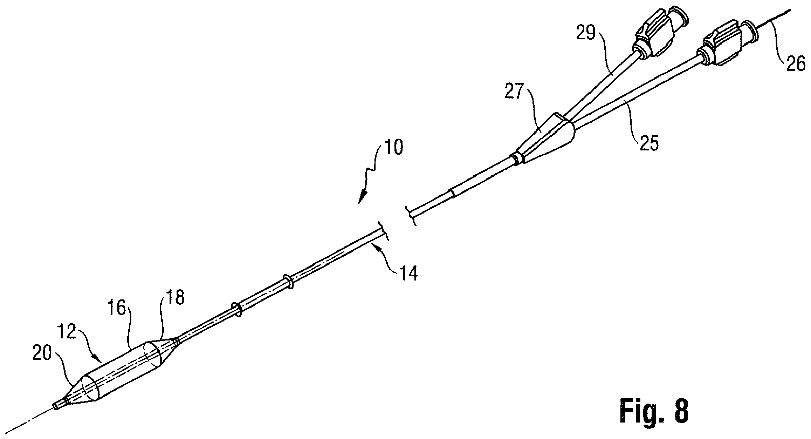

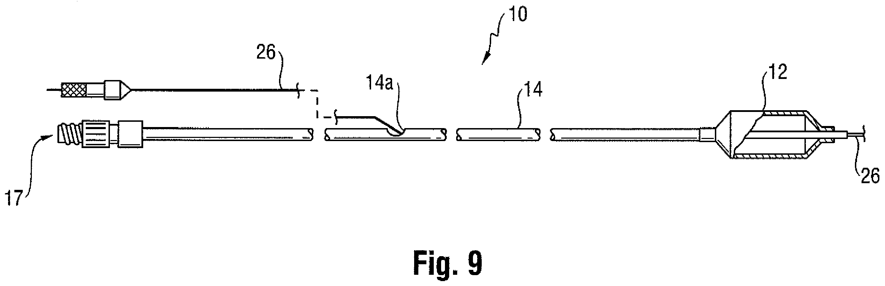

The catheter tube 14 also includes an elongated, tubular shaft 24 forming a guidewire lumen 23 that directs the guidewire 26 through the catheter 10, and along the distal end of which the balloon 12 may be located. As illustrated in Figure 8, this guidewire 26 may extend through the proximal end of the catheter 10 and a first port 25 of a connector 27 into the lumen 23 to achieve an "over the wire" (OTW) arrangement, but could also be provided in a "rapid exchange" (RX) configuration, in which the guidewire 26 exits a lateral opening 14a closer to the distal end (see Figure 9) or else is fed through the tip distally of the balloon 12 ("short" RX; see Figures 15 and 16). A second port 29 may also be associated with catheter 10, such as by way of connector 27, for introducing a fluid (e.g., saline, a contrast agent, or both) into the interior compartment of the balloon 12 via the inflation lumen 17.

Balloon 12 may include a single or multi-layered balloon wall 28 forming the interior for receiving the inflation fluid. The balloon 12 may be a non-compliant balloon having a balloon wall 28 that maintains its size and shape in one or more directions when the balloon is inflated. Examples of non-compliant balloons may be found in U.S. Pat. No. 6,746,425 and Publication Nos. US 2006/0085022, US 2006/0085023 and US 2006/0085024, The balloon 12 in such case also has a pre-determined surface area that remains constant during and after inflation, also has a pre-determined length and pre-determined diameter that each, or together, remain constant during and after inflation. However, the balloon 12 could be semi-compliant or compliant instead, depending on the particular use.

One embodiment for achieving the desired features is by arranging the shaft 24 in a manner that allows for it to expand, such as in the longitudinal direction, between a first or compressed condition having a nominal length to a second or expanded condition having a greater length than the nominal length. The expansion may be a relatively small amount, such as to overcome any misalignment that may otherwise be created (such as distance X). By choosing an appropriate arrangement, the expansion may be strategically controlled to help ensure that any radiopaque identifier provided, such as markers M, aligns with a particular portion of the balloon 12, such as the working surface W, once inflated.

With reference to Figure 10, this may be achieved in one embodiment by providing at least one expandable element 30 for allowing the shaft 24 to expand longitudinally, yet in a controlled manner, and thus ensure that the markers M remain in the desired alignment with a portion of the balloon 12. In one embodiment, the at least one expandable element 30 comprises a first spring 32a. The spring 32a may take the form of a helical or coil spring and, in particular, as a tension coil spring designed to elongate when placed in tension, such as the result of the expansion of the balloon 12 during inflation as a result of the connection at the distal tip P.

This spring 32a may positioned in tandem with a first, proximal portion 24a of the structure supporting the balloon 12, such as shaft 24 (to which the balloon 12 may be attached at one or both of the proximal and distal ends 15a, 15b), and another structure, such as an intermediate or second portion 24b of the shaft 24 in this particular embodiment. The attachment between the spring 32a and the corresponding shaft portion may be done by interference fit, embedding (possibly with a reduction in the stiffness of the shaft 24 to allow for increased flexibility despite the embedding), bonding (such as by adhesive or welding, either thermally or sonically). A second expandable element 30, such as spring 32b, may also be provided in tandem between the intermediate or second portion 24b and another structure, such as a distal or third portion 24c of the shaft 24. The shaft 24 as shown may include the radiopaque identifiers along one or more of the portions, such as intermediate portion 24b, in the form of spaced markers M aligned with the proximal and distal edges of the working surface W.

The springs 32a or 32b are selected to have a predetermined spring constant, k, to ensure the alignment of the markers M or other radiopaque identifiers in the desired manner. The spring constant is selected to correspond to the distensability of the balloon 12 on inflation. The shaft 24 as the result of the presence of springs 32a, 32b may expand a predetermined amount when the balloon 12 is inflated (see Figures 10a and 10b, and compare the length of shaft 24 corresponding to balloon 12 in the uninflated condition (12') and the inflated condition (12"), wherein L₁<L₂, and the springs 32a, 32b in the compressed 32a', 32b' and expanded or extended 32a", 32b" conditions, wherein Y₁<Y₂). This expansion, in turn, allows any radiopaque identifiers associated with the shaft 24, such as markers M on the intermediate portion 24b, to remain in alignment with the desired portion of the balloon 12, such as the working surface W (see dashed lines in Figure 10b). Consequently, the tendency for geographic miss may be reduced, since the clinician may place the markers M at the desired treatment location, where they will remain in alignment with and correspond to the full extent of the working surface W on inflation.

The expandable elements 30 may also be selected to provide different expansion characteristics during different stages of the inflation of the balloon 12. For example, as shown in Figure 11, a first spring 32a may have a lower spring constant, and a second spring 32b may have a higher spring constant. This permits the balloon 12 to experience some initial elastic deformation upon the commencement of inflation. The higher spring constant then allows for additional expansion of the support for the balloon 12, such as shaft 24, when higher inflation pressures are reached. Likewise, this higher spring constant allows the shaft 24 to return to an intermediate position when deflation begins.

When the springs comprise a radiopaque material, it is also possible that the different characteristics, such as the size or tightness of the winding, may allow for ready identification of different portions of the balloon 12. Thus, for example, the proximal spring 32a in Figure 11, if made radiopaque, would appear differently from the distal spring 32b, if also made radiopaque. This may aid the clinician in determining the orientation of the balloon 12 in an efficient manner.

This result may also be achieved using a single expandable element with variable expansion characteristics. For example, as shown in Figure 12, the hybrid spring 34 may include a loosely wound, higher radius portion 34a, and a more tightly wound, smaller radius portion 34b. Figure 11 also shows that the expandable elements 30, or springs 32a, 32b, may comprise, incorporate, carry, or otherwise include a radiopaque material, and thus serve as radiopaque identifiers in place of any marker bands or the like.

One or more of the expandable elements 30 may also form part of the tube 14 providing the inflation lumen 17 for inflating the balloon 12, and thus be independent of the shaft 24. For example, as shown in Figure 13, a first expandable element 30 in the form of a first spring 36a may extend between a first portion 14a of the tube 14 adjacent the proximal end 15a of the balloon 12 and a second portion 14b of the tube 14. A second expandable element 30 in the form of a second spring 36b may be positioned between the second portion 14b and a third portion 14c of the tube 14, adjacent to the distal end 15b of the balloon 12, adjacent to tip P. The springs 36a, 36b may be connected directly to the correspond ends of the tube portions 14a, 14b, 14c using adhesives or other bonding techniques, and as noted above may also be made of a radiopaque material to serve as markers for one or more portions of the balloon, such as the working surface W.

The balloon 12 at the tip P may be bonded in a manner such that it is connected to both the distal portion 14c and the shaft 24 forming the guidewire lumen 23. In such case, it should be appreciated that the relative expansion of the tube 14 cannot reliably or predictably occur without the relative movement of the shaft 24 in the longitudinal direction. One embodiment for achieving the desired relative movement is by arranging the shaft 24 in a manner that allows it to move a predetermined amount under a restrained condition in order to align the working surface W with particular location, such as the position of one or more radiopaque identifiers.

In this or another embodiment, as shown schematically in Figure 14, the catheter 100 may include a seal 102, such as may be provided by one or more O-rings or the like, at or near a proximal end of the shaft 24. The seal 102 is adapted for positioning in a recess 104 fonned in a receiver 101 for receiving the shaft 24, such as hub 106, which is shown as being oversized for purposes of illustration. The recess 104 is oversized so as to allow movement of the seal 102 and thus shaft 24 relative to the tube 14 within inflation lumen 17, which is in turn connected to the proximal end of the balloon 12. Other than at the distal end 15b of balloon 12 and the seal 102, the shaft 24 is not connected to any other structure in the catheter 10, and thus can move to and fro a distance in the longitudinal direction corresponding to the length of the recess 104, which will thus accommodate the relative expansion of the tube 14 as the result of the one or more expandable elements 30.

In this embodiment or others, it should also be appreciated that the expandable element 30 may serve as a port for allowing the inflation fluid to inflate the balloon 12. Specifically, the elements 30 in the form of springs 36a, 36b may expand during the initial pressurization of the tube 14 to allow the inflation fluid to enter the interior compartment of the balloon 12. The reverse arrangement could occur on deflation.

In an alternative embodiment, the expandable element(s) 30 may be used in connection without an inner shaft 24 for a guidewire, such as the "short" RX configurations shown in Figures 15 and 16. Specifically, a passage 49 at the tip P is adapted for receiving the guidewire. The tip P may comprise the distal end of a shaft 50 passing through the inner compartment of the balloon 12, which at the proximal end 15a associates with an inflation lumen 17.

The shaft 50 includes one or more expandable elements 30. For example, springs 38a, 38b may extend between proximal, intermediate, and distal portions 50a, 50b, 50b portions of the shaft 50, which may also include one or more radiopaque identifiers, such as markers M, corresponding to a portion of the balloon 12 such as the working surface W. On inflation, the desired relative expansion to ensure alignment of the identifiers in the intended manner is achieved by the selection of the spring constant to correspond to the distensibility of the balloon 12.

In this or another embodiment, the expandable element 30 may also be provided outside of the balloon 12, such as at the proximal end 15a. For example, Figure 16 shows a single expandable element 30 in the form of a spring 39 positioned between the tube 14 serving as the inflation lumen and the proximal end 15a of the balloon 12, where cone section 18 terminates. A sealing element 60 may seal the spring 39 to prevent leakage of the inflation fluid, such as by bridging from the distal end of the tube 14 to the balloon 12 (e.g., along the conical section 18 at the proximal end 15a). This arrangement thus allows the balloon 12 to move longitudinally as a result of the expansion of the element 30, or spring 39, to achieve alignment with the radiopaque identifiers, such as markers M.

Instead of being a separate component, as is contemplated above in at least one of the disclosed embodiments, or in addition to any such component, the expandable element 30 may also form part of the shaft 24 or 50. Thus, for example, as shown in Figure 17, the shaft 24 may be modified to include an expandable portion, which may comprise one or more helical, spring-like portions 40 corresponding to the working surface W, one or more flexible bellows portions 41 (Figure 18), or a fiber matrix that allows for a limited degree of expansion in the longitudinal direction (and possibly with an embedded spring 42 forming part of a mesh layer 25 of the shaft 24; see Figure 19).

The expandable elements may comprise metal materials, which provide ease of manufacturing, control over the spring constant, and, as noted above, radiopacity, if desired. A shape memory alloy, such as NITINOL, could also be used to promote a particular spring length in the expanded state as the result of a particular temperature (e.g., that of the body on which the procedure is performed, or of the inflation fluid). Alternatively, polymer materials with different elastic deformation properties could also be used as the expandable element (and may also be made radiopaque by embedding a radiopaque material in the polymer or coating the polymer).

Balloons 12 that carry one or more surface elements, such as a payload (drug, stent, or both) or a working implement (cutter, focused force wire, or the like) into the vasculature may also benefit from the foregoing description of marking techniques. For example, as shown in Figure 10, a balloon 12 including a defined working surface W may include a portion coated with such a drug D, such as one designed for achieving a desired therapeutic effect when applied to the interior of the vessel. The drug D may be applied to the inflated balloon as part of the manufacturing process, and prior to folding for insertion in the vasculature. The clinician may thus with the benefit of a fluoroscope determine the precise positioning of the working surface W prior to inflating the balloon 12 in the vasculature to deliver the drug D to the desired location and provide the desired treatment regimen.

## Claims

1. A balloon catheter, comprising:
a shaft (24; 50) extending in a longitudinal direction and adapted for expanding from a compressed condition to an expanded condition in the longitudinal direction,
the shaft supporting at least one radiopaque identifier, wherein the radiopaque identifier comprises a pair of spaced radiopaque markers (M), one positioned in alignment with a first end of the working surface (W) and another positioned at a second end of the working surface (W); and
an inflatable balloon (12) positioned along the shaft, the balloon when inflated including a working surface for aligning with the radiopaque identifier in at least the expanded condition of the shaft,
wherein the expandable shaft comprises a first portion connected in tandem to a spring as expandable element (30; 32a, 32b), wherein the spring constant is selected such that the relative expansion of the shaft corresponds to the distensibility of the balloon (12), so that the radiopaque identifiers and the working surface are aligned in the expanded condition.

2. The catheter of claim 1, wherein the expandable element comprises a spring, preferably comprising a coil spring, more preferably comprising a tension coil spring.

3. The catheter of claim 1, wherein the expandable element comprises a bellows (41).

4. The catheter of claim 1, wherein the expandable element comprises a fiber matrix.

5. The catheter of claim 4, further including a spring (42) associated with the fiber matrix.

6. The catheter of any of claims 2-5, wherein the expandable element is inside or outside an interior compartment of the balloon.

7. The catheter of any of claims 2-6, wherein the expandable element connects to one end of the balloon and/or connects the first portion of the shaft to a second portion of the shaft.

8. The catheter of any of the foregoing claims, wherein the shaft comprises an inflation lumen for delivering an inflation fluid to the balloon.

9. The catheter of any of the foregoing claims, wherein the expandable shaft serves in at least a partially expanded condition as a port for delivering the inflation fluid to the balloon, said port being closed when the shaft is in a non-expanded condition.

10. The catheter of any of the foregoing claims, wherein the expandable shaft comprises a first expandable element connecting a first portion of the shaft to a second portion of the shaft, and further including a second expandable element connecting the second portion of the shaft to a third portion of the shaft.

11. The catheter of claim 10, wherein the first and second expandable elements comprise first and second coil springs, preferably having different spring constants.

12. The catheter of any of claims 10-11, wherein the first and second expandable elements comprise a radiopaque material.

13. The catheter of any of the foregoing claims, wherein the radiopaque identifier comprises a spring.

14. The catheter of claim 1, wherein the expandable element comprises a spring having a variable spring constant.

15. The catheter of any of the foregoing claims, wherein the shaft comprises a guidewire lumen.

16. The catheter of any of the foregoing claims, further including a passage adjacent the tip for receiving a guidewire external to the balloon.

17. The catheter of claim 1, wherein the first portion is adjacent a distal end of the shaft.

## Patentansprüche

1. Ballonkatheter, umfassend:
einen Schaft (24; 50), der sich in einer Längsrichtung erstreckt und ausgelegt ist, sich von einem komprimierten Zustand in einen expandierten Zustand in der Längsrichtung zu expandieren,
wobei der Schaft mindestens eine röntgenopake Kennung trägt, wobei die röntgenopake Kennung ein Paar von beabstandeten röntgenopaken Markierungen (M) umfasst, von denen eine in Ausrichtung mit einem ersten Ende der Arbeitsfläche (W) und eine andere an einem zweiten Ende der Arbeitsfläche (W) angeordnet ist; und
einen aufblasbaren Ballon (12), der entlang des Schafts angeordnet ist, wobei der aufgeblasene Ballon eine Arbeitsfläche zum Ausrichten mit der röntgenopaken Kennung in zumindest dem expandierten Zustand des Schafts aufweist,
wobei der expandierbare Schaft einen ersten Abschnitt aufweist, der mit einer Feder als expandierbares Element (30; 32a, 32b) tandemverbunden ist, wobei die Federkonstante so gewählt ist, dass die relative Expansion des Schafts der Aufweitbarkeit des Ballons (12) entspricht, so dass die röntgenopaken Kennungen und die Arbeitsfläche in dem expandierten Zustand ausgerichtet sind.

2. Katheter nach Anspruch 1, wobei das expandierbare Element eine Feder umfasst, bevorzugt eine Schraubenfeder umfasst, bevorzugter eine Zugfeder umfasst.

3. Katheter nach Anspruch 1, wobei das expandierbare Element einen Balg (41) umfasst.

4. Katheter nach Anspruch 1, wobei das expandierbare Element eine Fasermatrix umfasst.

5. Katheter nach Anspruch 4, weiter aufweisend eine der Fasermatrix zugeordnete Feder (42).

6. Katheter nach einem der Ansprüche 2-5, wobei sich das expandierbare Element innerhalb oder außerhalb eines Innenraums des Ballons befindet.

7. Katheter nach einem der Ansprüche 2-6, wobei das expandierbare Element mit einem Ende des Ballons verbunden ist und/oder den ersten Teil des Schafts mit einem zweiten Teil des Schafts verbindet.

8. Katheter nach einem der vorstehenden Ansprüche, wobei der Schaft ein Aufblaslumen zur Abgabe einer Aufblasflüssigkeit an den Ballon umfasst.

9. Katheter nach einem der vorstehenden Ansprüche, wobei der expandierbare Schaft in zumindest teilweise expandiertem Zustand als eine Öffnung zum Zuführen der Aufblasflüssigkeit zum Ballon dient, wobei die Öffnung geschlossen ist, wenn sich der Schaft in einem nicht-expandierten Zustand befindet.

10. Katheter nach einem der vorstehenden Ansprüche, wobei der expandierbare Schaft ein erstes expandierbares Element umfasst, das einen ersten Abschnitt des Schafts mit einem zweiten Abschnitt des Schafts verbindet, und weiter ein zweites expandierbares Element aufweist, das den zweiten Abschnitt des Schafts mit einem dritten Abschnitt des Schafts verbindet.

11. Katheter nach Anspruch 10, wobei das erste und das zweite expandierbare Element erste und zweite Schraubenfedern umfassen, die vorzugsweise unterschiedliche Federkonstanten aufweisen.

12. Katheter nach einem der Ansprüche 10-11, wobei das erste und das zweite expandierbare Element ein röntgenopakes Material umfassen.

13. Katheter nach einem der vorstehenden Ansprüche, wobei die röntgenopake Kennung eine Feder umfasst.

14. Katheter nach Anspruch 1, wobei das expandierbare Element eine Feder mit einer variablen Federkonstante umfasst.

15. Katheter nach einem der vorstehenden Ansprüche, wobei der Schaft ein Führungsdrahtlumen umfasst.

16. Katheter nach einem der vorstehenden Ansprüche, weiter aufweisend einen Durchgang neben der Spitze zur Aufnahme eines Führungsdrahtes außerhalb des Ballons.

17. Katheter nach Anspruch 1, wobei der erste Teil an ein distales Ende des Schaftes angrenzt.

## Revendications

1. Cathéter à ballonnet, comprenant :
une tige (24; 50) s'étendant dans une direction longitudinale et adaptée pour se déployer d'un état comprimé à un état déployé dans la direction longitudinale,
la tige supportant au moins un identifiant radio-opaque, dans lequel l'identifiant radio-opaque comprend une paire de marqueurs radio-opaques espacés (M), l'un étant positionné en alignement avec une première extrémité de la surface de travail (W) et un autre étant positionné à une deuxième extrémité de la surface de travail (W) ; et
un ballonnet gonflable (12) positionné le long de la tige, le ballonnet lorsqu'il est gonflé incluant une surface de travail pour l'alignement avec l'identifiant radio-opaque dans au moins l'état déployé de la tige,
dans lequel la tige extensible comprend une première partie reliée en tandem à un ressort en tant qu'élément extensible (30, 32a, 32b), dans lequel la constante de ressort est sélectionnée de telle sorte que le déploiement relatif de la tige corresponde à la capacité de distension du ballonnet (12), de sorte que les identifiants radio-opaques et la surface de travail soient alignés à l'état déployé.

2. Cathéter selon la revendication 1, dans lequel l'élément extensible comprend un ressort, de préférence comprenant un ressort hélicoïdal, de manière davantage préférée, comprenant un ressort hélicoïdal à tension.

3. Cathéter selon la revendication 1, dans lequel l'élément extensible comprend un soufflet (41).

4. Cathéter selon la revendication 1, dans lequel l'élément extensible comprend une matrice à fibres.

5. Cathéter selon la revendication 4, incluant en outre un ressort (42) associé à la matrice à fibres.

6. Cathéter selon l'une quelconque des revendications 2 à 5, dans lequel l'élément extensible est à l'intérieur ou à l'extérieur d'un compartiment interne du ballonnet.

7. Cathéter selon l'une quelconque des revendications 2 à 6, dans lequel l'élément extensible est relié à une extrémité du ballonnet et/ou relie la première partie de la tige à une deuxième partie de la tige.

8. Cathéter selon l'une quelconque des revendications précédentes, dans lequel la tige comprend une lumière de gonflage pour délivrer un fluide de gonflage au ballonnet.

9. Cathéter selon l'une quelconque des revendications précédentes, dans lequel la tige extensible sert dans au moins un état partiellement déployé d'orifice pour délivrer le fluide de gonflage au ballonnet, ledit orifice étant fermé lorsque la tige est à l'état non déployé.

10. Cathéter selon l'une quelconque des revendications précédentes, dans lequel la tige extensible comprend un premier élément extensible reliant une première partie de la tige à une deuxième partie de la tige, et incluant en outre un deuxième élément extensible reliant la deuxième partie de la tige à une troisième partie de la tige.

11. Cathéter selon la revendication 10, dans lequel les premier et deuxième éléments extensibles comprennent des premier et deuxième ressorts hélicoïdaux, de préférence ayant différentes constantes de ressort.

12. Cathéter selon l'une quelconque des revendications 10 et 11, dans lequel les premier et deuxième éléments extensibles comprennent un matériau radio-opaque.

13. Cathéter selon l'une quelconque des revendications précédentes, dans lequel l'identifiant radio-opaque comprend un ressort.

14. Cathéter selon la revendication 1, dans lequel l'élément extensible comprend un ressort ayant une constante de ressort variable.

15. Cathéter selon l'une quelconque des revendications précédentes, dans lequel la tige comprend une lumière de fil-guide.

16. Cathéter selon l'une quelconque des revendications précédentes, incluant en outre un passage adjacent à l'embout pour recevoir un fil-guide externe au ballonnet.

17. Cathéter selon la revendication 1, dans lequel la première partie est adjacente à une extrémité distale de la tige.
